# EUROPÄISCHE PATENTSCHRIFT

(11) **EP 1 116 477 B1**
(45) Veröffentlichungstag und Bekanntmachung des Hinweises auf die Patenterteilung: **03.05.2006**
(21) Anmeldenummer: 00120737.2
(22) Anmeldetag: 22.09.2000
(51) Int. Cl.: A61B 19/00, A61B 17/00

(54) **Chirurgische Werkzeugstation**
Surgical tool station
Station à outils chirurgicaux

(30) Priorität: 22.09.1999 DE 19945208
(43) Veröffentlichungstag der Anmeldung: 18.07.2001
(73) Patentinhaber: Adrian, Ute, 10439 Berlin (DE)
(72) Erfinder: Adrian, Ute, 10439 Berlin (DE)
(74) Vertreter: Niederkofler, Oswald

(56) Entgegenhaltungen:
- EP-A- 0 548 031
- DE-U- 8 621 578
- US-A- 4 705 038
- US-A- 4 936 842
- US-A- 5 871 493

## Beschreibung

Die Erfindung betrifft eine chirurgische Werkzeugstation gemäß dem Oberbegriff des Patentanspruchs 1.

Eine derartige chirurgische Werkzeugstation ist bereits aus der US 4 705 038 bekannt. Diese Werkzeugstation weist eine Steuer- und Antriebseinheit auf, an der Anschlüsse für verschiedene Werkzeuge und ein Drehschalter angeordnet sind, der zum Auswählen eines dieser Anschlüsse und somit des daran angeschlossenen Werkzeugs dient. Ein Fußschalter, der an die Steuer- und Antriebseinheit angeschlossen ist, dient zum Ein- und Ausschalten des mit dem Drehschalter ausgewählten Werkzeugs.

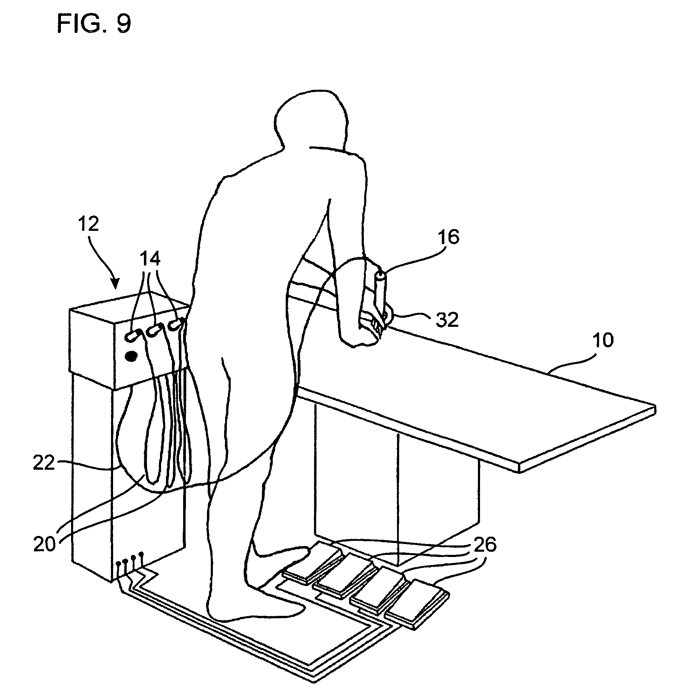

Die FIG. 9 zeigt schematisch eine andere bekannte chirurgische Werkzeugstation 12 neben einem Operationstisch 10. Die dargestellte Werkzeugstation 12 weist vier Werkzeuge 14, 16 auf. Die mit Hilfsenergie betriebenen Werkzeuge umfassen beispielsweise Laserskalpelle 16, aber auch solche, die elektrisch, pneumatisch oder über flexible Wellen 20 angetrieben werden, wie zum Beispiel Kreis- und Stichsägen, Fräsen, Stanzen, Schleifteller, Bohrmaschinen, Band- und Bohrschleifer.

Die bekannte chirurgische Werkzeugstation 12 weist außerdem zu jedem Werkzeug 14, 16 einen eigenen Fußschalter 26 zum Ein- und Ausschalten dieses Werkzeugs 14, 16 auf. Die verschiedenen Fußschalter 26 sind dabei unter dem Operationstisch 10 angeordnet. Daher kann der Chirurg, wenn er sich, wie in der FIG. 9 dargestellt ist, über den Operationstisch 10 beugt, die Fußschalter 26 nicht mehr sehen, so dass die Gefahr besteht, dass er die Fußschalter 26 verwechselt und versehentlich nicht das Werkzeug 16 einschaltet, das er gerade in der Hand 32 hat, sondern eines der anderen Werkzeuge 14 einschaltet, die er gerade nicht benötigt. Dadurch kann es zu erheblichen Verletzungen kommen, wenn zum Beispiel die assistierende Schwester eines dieser gerade nicht benötigten Werkzeuge 14 reinigt und dieses dann versehentlich von dem Chirurg eingeschaltet wird.

Es ist daher Aufgabe der Erfindung, eine chirurgische Werkzeugstation der eingangs genannten Art zur Verfügung zu stellen, die das Risiko vermindert, dass der Chirurg versehentlich nicht das gerade von ihm gehaltene Werkzeug, sondern eines der anderen Werkzeuge einschaltet.

Diese Aufgabe wird durch die chirurgische Werkzeugstation gemäß dem Patentanspruch 1 gelöst.

Gemäß der Erfindung kann ein bestimmtes Werkzeug nur dann mit dem Hauptschalter eingeschaltet werden, wenn auch der Wahlschalter derart betätigt ist, dass dieses bestimmte Werkzeug ausgewählt wird. Da nur noch ein Hauptschalter vorhanden ist, kann sich der Chirurg voll und ganz auf seine Tätigkeit und das unmittelbare Umfeld um die Ansatzstelle des von ihm gerade geführten Werkzeugs konzentrieren. Das Risiko für das assistierende Personal aufgrund einer Verwechslung verschiedener Fußschalter ist beseitigt.

Vorteilhafte Weiterbildungen sind in den Unteransprüchen beschrieben.

Es ist vorgesehen, dass jedes Werkzeug einen eigenen Wahlschalter aufweist, der einen berührungslosen Näherungssensor aufweist, der mit einem Erkennungselement zusammenarbeitet. Dieses Erkennungselement wird vorzugsweise an derjenigen Hand des Chirurgen angebracht, die das Werkzeug hält. Wenn er ein bestimmtes Werkzeug in diese Hand nimmt, dann befindet sich das Erkennungselement im Ansprechbereich des Näherungssensors, wodurch der Wahlschalter an diesem Werkzeug betätigt wird und die Steuereinheit dieses ausgewählte Werkzeug freigibt. Wenn der Chirurg dieses Werkzeug aus der Hand legt und beispielsweise der assistierenden Schwester überreicht, dann gerät das Erkennungselement aus dem Ansprechbereich des Näherungssensors, wodurch der Wahlschalter dieses Werkzeugs umgeschaltet wird und die Steuereinheit dieses Werkzeug sperrt.

Der Näherungssensor kann beispielsweise vom induktiven Typ sein. In diesem Fall ist das Erkennungselement elektrisch leitfähig, um den induktiven Näherungssensor bei Annäherung nach Wunsch beeinflussen zu können. Das Erkennungselement kann beispielsweise ein Metallring, den der Chirurg an einem Finger oder dem Handgelenk seiner die Werkzeuge führenden Hand trägt, oder auch eine Metallplakette sein, die auf den Handrücken der die Werkzeuge führenden Hand geklebt wird oder an dem Einweghandschuh für diese Hand angebracht ist. In diesem Fall sollten die assistierenden Schwestern, die ebenfalls die Werkzeuge in die Hand nehmen müssen, keine elektrisch leitenden Objekte an ihren Händen aufweisen, damit ausgeschlossen werden kann, dass sie versehentlich ein Werkzeug freigeben.

Es kann auch vorgesehen sein, dass das Erkennungselement einen Sender zur Abstrahlung eines Kennsignals und der Näherungssensor einen Empfänger für das Kennsignal aufweist.

In diesem Fall ist bevorzugt vorgesehen, dass der Näherungssensor einen Sender zur Abstrahlung eines Abfragesignals und das Erkennungselement einen Transponder aufweist, der so ausgebildet ist, dass er das Kennsignal nur dann abstrahlt, wenn er das Abfragesignal empfängt.

Es ist auch möglich, dass der Wahlschalter an der Steuereinheit angeordnet ist, und dass jedes Werkzeug eine Signalleuchte aufweist, die nur dann von der Steuereinheit angesteuert wird, wenn das ihr zugeordnete Werkzeug durch den Wahlschalter ausgewählt ist. Bei dieser Variante wird das gewünschte Werkzeug dadurch ausgewählt, dass beispielsweise die assistierende Schwester den Wahlschalter betätigt. Sie hat daher direkten Einfluss auf ihre eigene Sicherheit, da es in ihrem eigenen Interesse liegt, das vom Chirurgen gewünschte Werkzeug erst dann auszuwählen bzw. freizugeben, wenn sie es ihm überreicht hat. Die Signalleuchte dient ihr dabei als Rückmeldung dafür, dass sie das richtige Werkzeug ausgewählt hat.

Es kann auch vorgesehen sein, dass jedes Werkzeug einen eigenen Wahlschalter aufweist, der im Griffbereich des Werkzeugs angeordnet ist. Dieser Wahlschalter wird daher betätigt, wenn der Chirurg das Werkzeug ordnungsgemäß in seiner Hand hält. Aus Sicherheitsgründen sollte dann darauf geachtet werden, dass die assistierende Schwester bei der Handhabung dieses Werkzeugs darauf achtet, den Griffbereich und vor allem den Wahlschalter zu meiden.

Bevorzugt ist der Hauptschalter ein Fußschalter.

Im folgenden werden bevorzugte Ausführungsformen der Erfindung beispielhaft anhand der beigefügten Zeichnungen beschrieben.
- FIG. 1: ist eine perspektivische Gesamtansicht, die eine chirurgische Werkzeugstation in einer ersten Ausführungsform zeigt;
- FIG. 2: ist eine perspektivische Gesamtansicht, die eine chirurgische Werkzeugstation in einer zweiten Ausführungsform zeigt;
- FIG. 3: ist eine Detailansicht, die ein Werkzeug mit einem eigenen Wahlschalter in einer ersten Ausführungsform zeigt;
- FIG. 4: ist eine Detailansicht, die das Werkzeug der FIG. 3 mit einem anderen Erkennungselement zeigt;
- FIG. 5: ist eine Detailansicht, die ein Werkzeug mit einem eigenen Wahlschalter in einer zweiten Ausführungsform zeigt;
- FIG. 6: ist eine Detailansicht, die das Werkzeug der FIG. 5 und eine alternative Plazierung des Transponders zeigt;
- FIG. 7: ist eine Detailansicht, die ein Werkzeug mit einem eigenen Wahlschalter in einer dritten Ausführungsform, die keinen Teil der Erfindung ausmacht, zeigt;
- FIG. 8: ist eine Detailansicht, die ein Werkzeug mit einem eigenen Wahlschalter in einer vierten Ausführungsform, die keinen Teil der Erfindung ausmacht, zeigt;
- FIG. 9: ist eine perspektivische Gesamtdarstellung, die eine bekannte chirurgische Werkzeugstation zeigt.

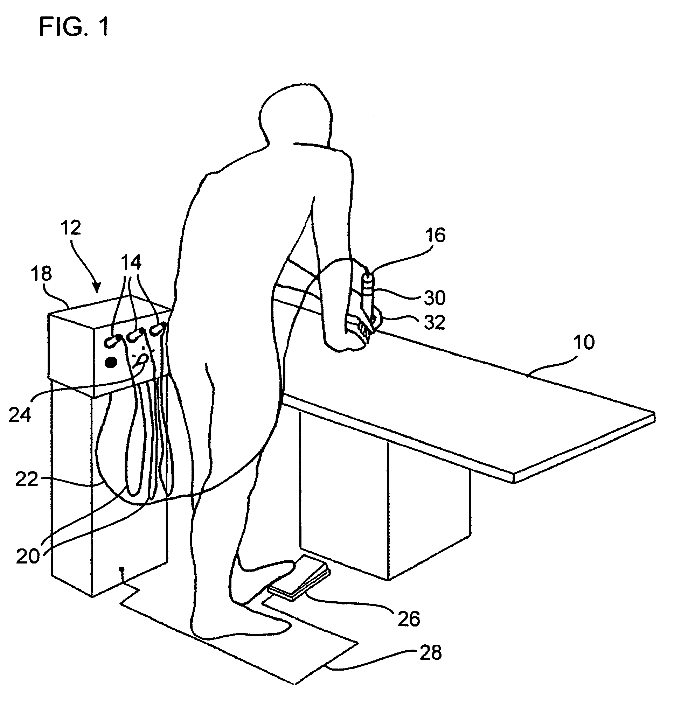

Die FIG. 1 zeigt einen Operationstisch 10 und eine chirurgische Werkzeugstation 12, die vier mit Hilfsenergie betriebene Werkzeuge 14, 16, eine Schalteranordnung zum selektiven Ein- und Ausschalten der Werkzeuge 14, 16 und eine Steuereinheit 18 aufweist. Die Werkzeuge umfassen drei über flexible Wellen 20 angetriebene Werkzeuge 14, die beispielsweise eine Fräse, eine Kreissäge und ein Bohrer sind, sowie ein Laserskalpell 16, das über ein Lichtleiterkabel 22 mit der Laserquelle (nicht dargestellt) verbunden ist.

Bei dieser ersten Ausführungsform der Werkzeugstation 12 die keinen Teil der Erfindung ausmacht weist die Schalteranordnung einen Drehschalter 24 mit fünf Raststellungen, der am Gehäuse der Steuereinheit 18 angebracht ist, und einen Fußschalter 26 auf. Der Fußschalter 26 ist so unterhalb des Operationstisches 10 angeordnet, dass ein Chirurg diesen bequem betätigen kann, wenn er sich über den Operationstisch 10 beugt. Der Fußschalter 26 ist über ein Kabel 28 mit der Steuereinheit 18 verbunden.

Der Drehschalter 24 dient als Wahlschalter zum Auswählen eines der Werkzeuge 14, 16. Im folgenden wird beispielhaft angenommen, dass das Laserskalpell 16 das ausgewählte Werkzeug ist und die über Wellen 20 angetriebenen Werkzeuge 14 die nicht ausgewählten Werkzeuge sind. Der Drehschalter 24 ist derart mit der Steuer einheit 18 verbunden, dass vier der fünf Raststellungen des Drehschalters 24 jeweils einem der vier Werkzeuge 14, 16 zugeordnet sind (und im folgenden auch als Auswahlstellungen bezeichnet werden) und die fünfte Raststellung die AUS-Stellung ist. Die Steuereinheit 18 ist so ausgebildet, dass sie, falls sich der Drehschalter 24 in der AUS-Stellung befindet, sämtliche Werkzeuge 14, 16 sperrt und, falls sich der Drehschalter 24 in einer der vier Auswahlstellungen befindet, dasjenige Werkzeug 14, 16 freigibt, dem diese Auswahlstellung zugeordnet ist. Hierbei wird unter "Freigeben eines ausgewählten Werkzeugs" verstanden, dass die Steuereinheit 18 weiter so ausgebildet ist, dass sie dieses ausgewählte Werkzeug mit seiner Hilfsenergie versorgt, wenn der Fußschalter 26 betätigt wird. Der Fußschalter 26 wird also von der Steuereinheit 18 als Hauptschalter zum Ein- und Ausschalten des ausgewählten Werkzeugs 14, 16 geschaltet. Die übrigen Werkzeuge 14 lassen sich in dieser Auswahlstellung nicht mit dem Fußschalter 26 in Betrieb setzen, sie sind gesperrt. In der AUS-Stellung sind sämtliche Werkzeuge 14, 16 gesperrt.

An jedem Werkzeug 14, 16 ist eine Signalleuchte 30 vorgesehen, die nur dann von der Steuereinheit 18 in Betrieb gesetzt wird, wenn das ihr zugeordnete Werkzeug 14, 16 durch den Drehschalter 24 ausgewählt worden ist. Die Signalleuchten 30 geben daher den Freigabezustand der Werkzeuge 14, 16 an, so dass die assistierende Schwester darauf achten kann, dass sie nur das Werkzeug 16 mit der leuchtenden Signalleuchte 30 dem Chirurg überreicht.

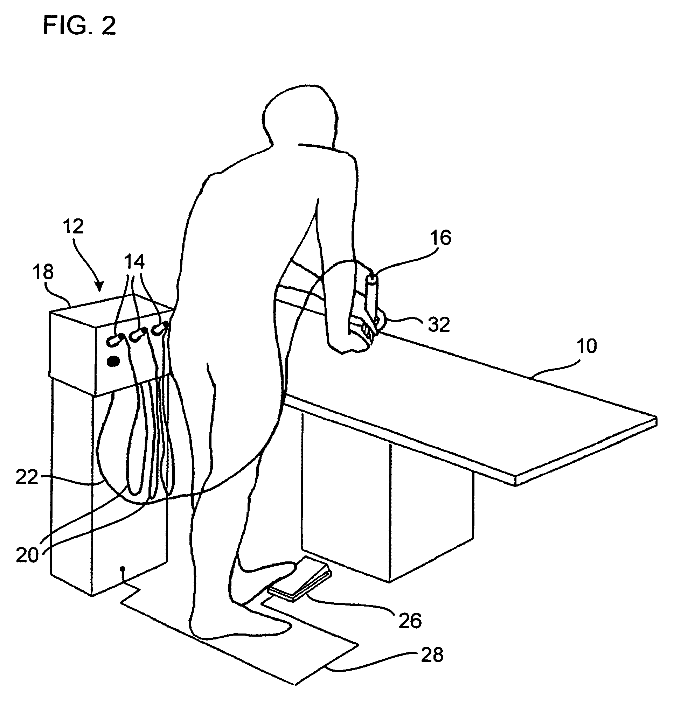

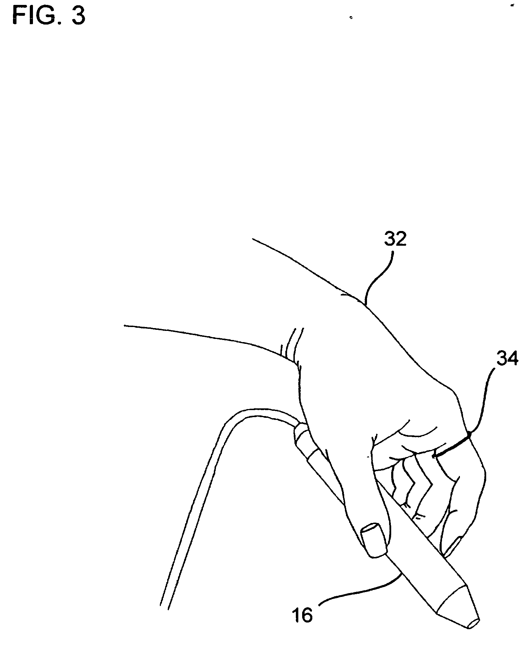

Die FIG. 2 zeigt eine chirurgische Werkzeugstation 12 in einer zweiten Ausführungsform, die sich von der ersten Ausführungsform der FIG. 1 durch die Ausgestaltung der Schalteranordnung zum selektiven Ein- und Ausschalten der Werkzeuge 14, 16 unterscheidet. Anstelle des bei der ersten Ausführungsform vorgesehenen Drehschalters 24 an der Steuereinheit 18 ist bei dieser zweiten Ausführungsform an jedem Werkzeug 14, 16 ein eigener Wahlschalter (nicht dargestellt) vorgesehen. Diese Wahlschalter sind derart ausgebildet, dass ein Werkzeug 16 dadurch ausgewählt werden kann, dass der Chirurg dieses Werkzeug 16 in die Hand nimmt und den Wahlschalter dieses Werkzeugs 16 betätigt. Als Wahlschalter kommen verschiedene Möglichkeiten in Betracht, von denen einige im folgenden anhand der FIG. 3 bis 8 beschrieben werden.

Die FIG. 3 zeigt die Hand 32 des Chirurgen, die ein ausgewähltes Werkzeug 16 hält, das einen Wahlschalter (nicht dargestellt) in einer ersten Ausführungsform aufweist. Dieser Wahlschalter ist ein berührungsloser Näherungssensor, der bei dieser ersten Ausführungsform als induktiver Näherungssensor ausgebildet ist. Da induktive Näherungssensoren an sich im Stand der Technik bekannt sind, wird hier auf eine Beschreibung ihres Aufbaus und ihrer Funktionsweise verzichtet.

Der Näherungssensor arbeitet mit einem Erkennungselement zusammen, das im Hinblick auf den induktiven Näherungssensor elektrisch leitfähig ist und hier als Metallring 34 vorliegt, den der Chirurg am Zeigefinger seiner das ausgewählte Werkzeug 16 haltenden Hand 32 trägt. Der induktive Näherungssensor ist in dem Gehäuse des Werkzeugs 16 angeordnet und derart eingestellt, dass er dieses Werkzeug 16 freigibt, wenn es der Chirurg in seiner Hand 32 hält und somit der Ring 34 sich im Ansprechbereich des Sensors befindet. Sobald der Chirurg dieses Werkzeug 16 aus der Hand 32 legt und beispielsweise der assistierenden Schwester übergibt, wird auch der Ring 34 aus dem Ansprechbereich des induktiven Näherungssensors entfernt, so dass dieser das ihm zugeordnete Werkzeug 16 sperrt.

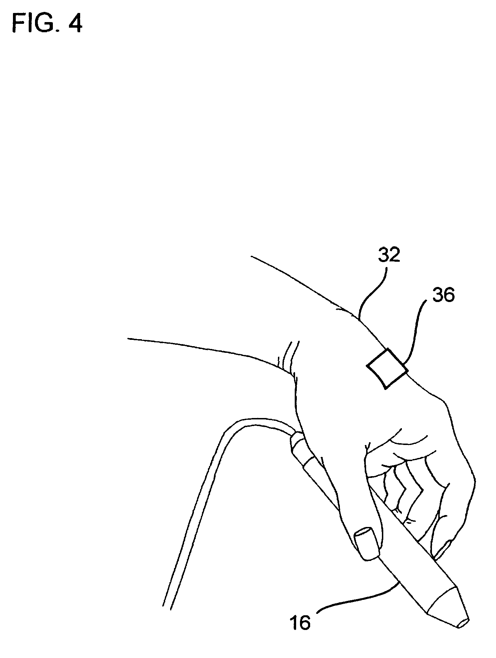

Die FIG. 4 zeigt das Werkzeug 16 der FIG. 3, jedoch ist hier als Erkennungselement für den induktiven Näherungssensor anstelle eines Ringes 34 eine Metallplakette 36 vorgesehen, die auf den Handrücken der das Werkzeug 16 haltenden Hand 32 des Chirurgen geklebt ist.

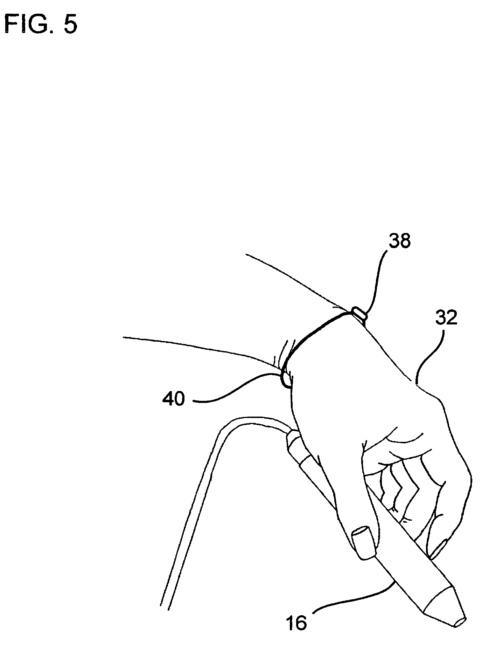

Die FIG. 5 zeigt ein ausgewähltes Werkzeug 16 mit einem eigenen Wahlschalter in einer zweiten Ausführungsform. Auch hier ist der Wahlschalter ein berührungsloser Näherungssensor (nicht dargestellt), der mit einem Erkennungselement zusammenarbeitet. Bei dieser zweiten Ausführungsform weist der Näherungssensor einen Sender zur Abstrahlung eines Abfragesignals und einen Empfänger für ein Kennsignal auf. Das Erkennungselement 38 weist einen Transponder (nicht dargestellt) auf, der einen Empfänger für das Abfragesignal, einen Sender zur Abstrahlung des Kennsignals und eine Steuerschaltung aufweist. Dieses Steuerschaltung ist derart ausgebildet, dass der Transponder das Kennsignal nur dann abstrahlt, wenn er das Abfragesignal empfängt. Das Erkennungselement 38 mit dem Transponder ist hier an einem Armband 40 befestigt, das der Chirurg am Handgelenk seiner das Werkzeug 16 haltenden Hand 32 trägt.

Die Sendeleistungen des Abfragesignal-Senders und des Kennsignal-Senders und die Empfangsempfindlichkeiten des Abfragesignal-Empfängers und des Kennsignal-Empfängers definieren den Ansprechbereich dieses berührungslosen Näherungssensors und sind so gewählt, dass, wie schon weiter oben bei der FIG. 3 erläutert, das Werkzeug 16 freigegeben ist, wenn es der Chirurg in seiner Hand 32, also in der Nähe des Transponders hält, dass es hingegen gesperrt ist, sobald er es aus dieser Hand 32 legt und beispielsweise eines der anderen, bisher gesperrten Werkzeuge 14 in die Hand 32 nimmt.

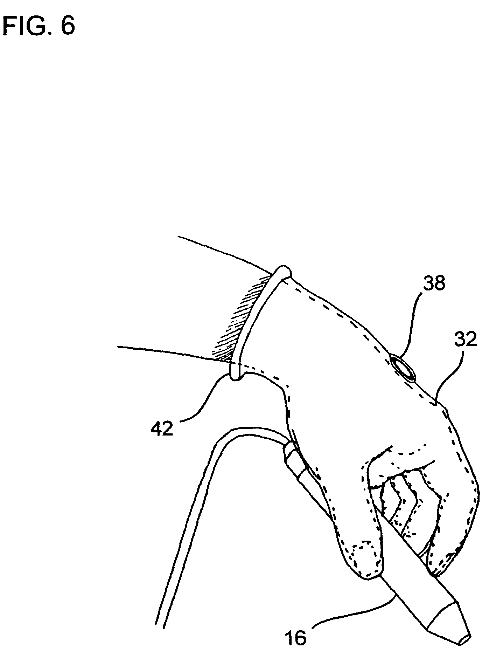

Die FIG. 6 zeigt das Werkzeug 16 der FIG. 5, jedoch ist hier das Erkennungselement 38 mit dem Transponder an einem Einweghandschuh 42 angebracht.

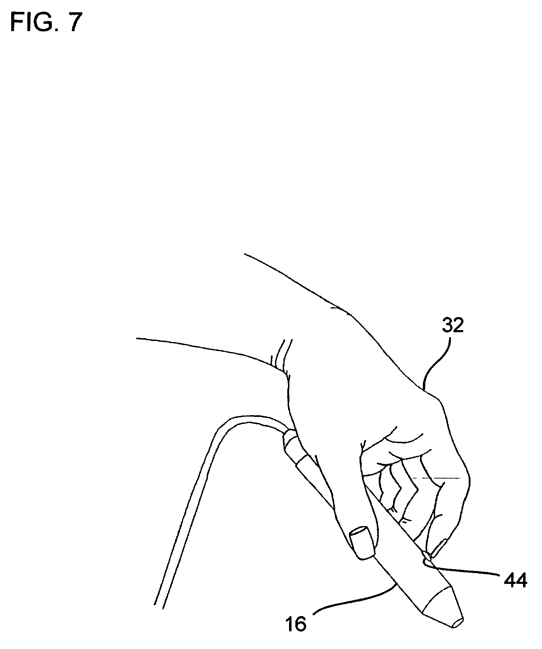

Die FIG. 7 zeigt ein ausgewähltes Werkzeug 16, das einen Wahlschalter in einer dritten Ausführungsform, die keinen Teil der Erfindung ausmacht, aufweist. Der Wahlschalter ist hier ein Drucktaster 44, der im Griffbereich des Werkzeugs 16 derart angeordnet ist, dass er von dem Chirurgen bequem betätigt werden kann, wenn er das Werkzeug 16 benutzt. Gemäß der FIG. 7 ist der Drucktaster 44 beispielsweise unter dem Zeigefinger der Hand 32 angeordnet.

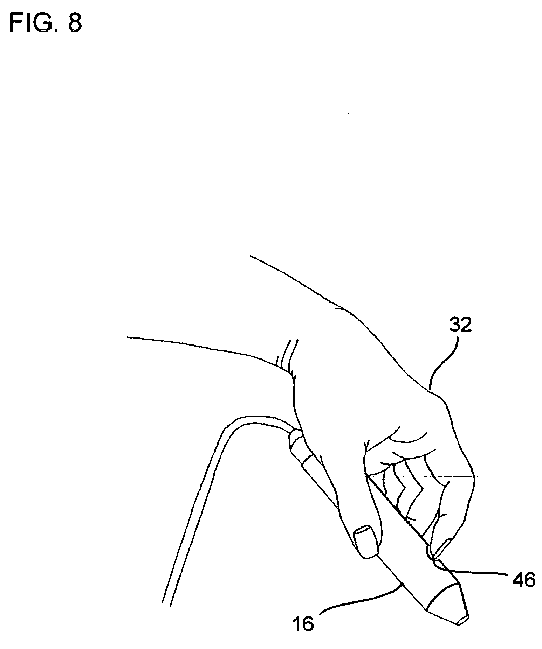

Die FIG. 8 zeigt ein ausgewähltes Werkzeug 16, das einen Wahlschalter in einer vierten Ausführungsform, die keinen Teil der Erfindung ausmacht, aufweist. Bei dieser vierten Ausführungsform ist der Drucktaster 44 der FIG. 7 durch eine Lichtschranke (nicht dargestellt) ersetzt, die ebenfalls im Griffbereich des Werkzeugs 16 angeordnet ist. Gemäß der FIG. 8 weist das Gehäuse des Werkzeugs 16 unter der Fingerspitze des Zeigefingers der Hand 32 eine Vertiefung 46 auf. Innerhalb des Gehäuses sind der Sender und der Empfänger der Lichtschranke derart angeordnet, dass die nach innen gewölbte Wand der Vertiefung 46 zwischen ihnen liegt. Diese Wand ist transparent ausgebildet, so dass das von dem Sender ausgestrahlte Licht durch die Wand auf den Empfänger treffen kann. Dieser Lichtstrahl wird unterbrochen, wenn die Spitze des Zeigefingers, wie in der FIG. 8 dargestellt, in der Vertiefung ruht.

## Patentansprüche

1. Chirurgische Werkzeugstation, mit:
- wenigstens zwei mit Hilfsenergie betriebenen Werkzeugen (14, 16);
- einer Schalteranordnung (26) zum selektiven Ein- und Ausschalten der Werkzeuge (14, 16), die wenigstens einen Wahlschalter zum Auswählen eines Werkzeugs (16) und einen Hauptschalter (26) zum Ein- und Ausschalten dieses ausgewählten Werkzeugs (16) aufweist;
- einer Steuereinheit (18), die bei Betätigung der Schalteranordnung (26; 44, 26) die Hilfsenergie an ein ausgewähltes Werkzeug (16) leitet,
**dadurch gekennzeichnet, dass**
- jedes Werkzeug (14, 16) einen eigenen Wahlschalter aufweist, der einen berührungslosen Näherungssensor aufweist; und
- der Näherungssensor mit einem Erkennungselement (34, 36, 38) zusammenarbeitet; das vom Anwender an der Hand getragen wird.

2. Werkzeugstation nach Anspruch 1, **dadurch gekennzeichnet, dass** der Näherungssensor einen induktiven Näherungssensor aufweist, und dass das Erkennungselement (34, 36) elektrisch leitfähig ist.

3. Werkzeugstation nach Anspruch 1, **dadurch gekennzeichnet, dass** das Erkennungselement (38) einen Sender zur Abstrahlung eines Kennsignals und der Näherungssensor einen Empfänger für das Kennsignal aufweist.

4. Werkzeugstation nach Anspruch 3, **dadurch gekennzeichnet, dass** der Näherungssensor einen Sender zur Abstrahlung eines Abfragesignals und das Erkennungselement (38) einen Transponder aufweist, der so ausgebildet ist, dass er das Kennsignal nur dann abstrahlt, wenn er das Abfragesignal empfängt.

5. Werkzeugstation nach einem der vorhergehenden Ansprüche, **dadurch gekennzeichnet, dass** jedes Werkzeug (14, 16) einen eigenen Wahlschalter (44) aufweiset, der im Griffbereich des Werkzeugs (14, 16) angeordnet ist.

6. Werkzeugstation nach einem der vorhergehenden Ansprüche, **dadurch gekennzeichnet, dass** der Hauptschalter ein Fußschalter (26) ist.

## Revendications

1. Station à outils chirurgicaux, comprenant:
- au moins deux outils (14, 16) actionnés par servo-énergie;
- un arrangement (26) de commutateurs pour sélectivement mettre en marche et mettre hors marche les outils (14, 16), l'arrangement (26) de commutateurs comprenant au moins un commutateur de sélection pour la sélection d' un outil (16) et un commutateur général (26) pour mettre en marche et mettre hors marche cet outil (16) sélectionné; et
- une unité de commande(18) qui dirige le servo-énergie à un outil (16) sélectionné en cas de mise en action de 1' arrangement (26) de commutateurs,
**caractérisée en ce que**
- chaque outil (14, 16) comprend son propre commutateur de sélection qui comprend un détecteur de proximité sans contact; et
- le détecteur de proximité coopère avec un élément d' identité (34, 36, 38) qui est porté par l'usager à la main.

2. Station à outils selon la revendication 1, **caractérisée en ce que** le détecteur de proximité comprend un détecteur de proximité inductif, et **en ce que** l'élément d' identité (34, 36) est électriquement conductible.

3. Station à outils selon la revendication 1, **caractérisée en ce que** l'élément d' identité (38) comprend un émetteur pour la émission d'un signal d' identification, et **en ce que** le détecteur de proximité comprend un récepteur pour le signal d' identification.

4. Station à outils selon la revendication 3, **caractérisée en ce que** le détecteur de proximité comprend un émetteur pour la émission d'un signal d' interrogation, et **en ce que** l'élément d' identité (38) comprend un répondeur qui est formé à tel point qu' il émet le signal d' identification seulement dans le cas où il reçoit le signal d' interrogation.

5. Station à outils selon une des revendications précédentes, **caractérisée en ce que** chaque outil (14, 16) comprend son propre commutateur de sélection (44) qui est disposé dans la zone de la manche de l'outil (14, 16).

6. Station à outils selon une des revendications précédentes, **caractérisée en ce que** le commutateur général est un commutateur (26) à commande au pied.

## Claims

1. Surgical tool station, comprising:
- at least two tools (14, 16) driven by servo energy;
- an arrangement (26) of switches for selectively connecting and disconnecting the tools (14, 16), the arrangement (26) of switches comprising at least one selection switch for selecting a tool (16), and a main switch (26) for connecting and disconnecting said selected tool (16); and
- a control unit (18) which directs the servo energy to a selected tool (16) if the arrangement (26) of switches is actuated,
**characterized in that**
- each tool (14, 16) comprises its own selection switch which comprises a contactless proximity sensor; and
- the proximity sensor cooperates with an identification element (34, 36, 38) which is worn by the user on the hand.

2. Tool station according to claim 1, **characterized in that** the proximity sensor comprises an inductive proximity sensor, and **in that** the identification element (34, 36) is electrically conductive.

3. Tool station according to claim 1, **characterized in that** the identification element (38) comprises a transmitter for radiating an identification signal, and **in that** the proximity sensor comprises a receiver for the identification signal.

4. Tool station according to claim 3, **characterized in that** the proximity sensor comprises a transmitter for radiating an inquiry signal, and **in that** the identification element (38) comprises a transponder which is designed in such a manner that it radiates the identification signal only if it receives the inquiry signal.

5. Tool station according to one of the preceding claims, **characterized in that** each tool (14, 16) comprises its own selection switch (44) which is disposed in the handle region of the tool (14, 16).

6. Tool station according to one of the preceding claims, **characterized in that** the main switch is a foot switch (26).
